(19) Europäisches Patentamt
European Patent Office
Office européen des brevets

(11) Veröffentlichungsnummer: 0 089 597
A2

(12) EUROPÄISCHE PATENTANMELDUNG

(21) Anmeldenummer: 83102522.6

(22) Anmeldetag: 15.03.83

(51) Int. Cl.[3]: C 07 D 215/54
A 61 K 31/64

(30) Priorität: 19.03.82 DE 3210063

(43) Veröffentlichungstag der Anmeldung: 28.09.83 Patentblatt 83/39

(84) Benannte Vertragsstaaten: AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Hitzel, Volker. Dr.
Kantstrasse 1b
D-6238 Hofheim am Taunus(DE)

(72) Erfinder: Weyer, Rudi, Dr.
Mozartstrasse 8
D-6233 Kelkheim (Taunus)(DE)

(72) Erfinder: Geisen, Karl, Dr.
Jahnstrasse 43
D-6000 Frankfurt am Main(DE)

(72) Erfinder: Ritzel, Harald, Dr.
An der Hayle 4
D-6500 Mainz(DE)

(54) **Sulfonylharnstoffe, Verfahren zu ihrer Herstellung, pharmazeutische Präparate auf Basis dieser Verbindungen und ihre Verwendung.**

(57) Sulfonylharnstoffe der Formel

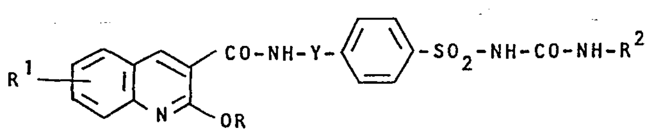

worin R, $R^1$, $R^2$ und Y die angegebenen Bedeutungen haben und deren physiologisch verträgliche Salze, Verfahren zu ihrer Herstellung, pharmazeutische Präparate auf Basis dieser Verbindungen sowie ihre Verwendung als Antidiabetika.



Croydon Printing Company Ltd.

HOECHST AKTIENGESELLSCHAFT HOE 82/F 052 Dr.D/Pa

Sulfonylharnstoffe, Verfahren zu ihrer Herstellung, pharmazeutische Präparate auf Basis dieser Verbindungen und ihre Verwendung

Die Erfindung betrifft Sulfonylharnstoffe der Formel

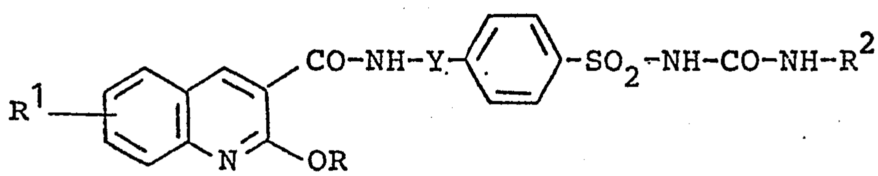

die als Substanz oder in Form ihrer physiologisch verträglichen Salze blutzuckersenkende Eigenschaften besitzen, sich durch eine starke Senkung des Blutzuckerspiegels auszeichnen und daher als Arzneimittel verwendet werden können.

In der Formel bedeuten:

Y Alkylen mit 2 - 3 C-Atomen,
R Alkyl mit 1 - 8 C-Atomen, Cycloalkyl oder Alkylcycloalkyl mit jeweils 5 - 8 C-Atomen, Phenyl, das gegebenenfalls durch Alkyl mit 1 - 4 C-Atomen, Alkoxy mit 1 - 4 C-Atomen im Alkylteil oder Halogen substituiert ist,
$R^1$ Wasserstoff, Halogen oder Alkyl mit 1 - 4 C-Atomen,
$R^2$ Alkyl mit 3 - 6 C-Atomen, Cycloalkyl, Alkylcycloalkyl, Cycloalkylalkyl, Cycloalkenyl, Alkylcycloalkenyl oder Cycloalkenylalkyl mit jeweils 5 - 8 C-Atomen.

Halogen bedeutet Chlor und Brom, wobei Chlor bevorzugt ist.
Y steht vorzugsweise für $-CH_2-CH_2-$ oder $-CH(CH_3)-CH_2-$, wobei die $-CH_2-CH_2-$Gruppe besonders bevorzugt ist.
R in der Bedeutung Alkyl steht vorzugsweise für Alkylgruppen mit 1 - 5 C-Atomen. Für R in der Bedeutung Cycloalkyl ist Cyclohexyl besonders bevorzugt und für R in der Bedeutung Phenyl ist das unsubstituierte Phenyl die bevorzugte Gruppierung.

$R^1$ bedeutet vorzugsweise Wasserstoff und $R^2$ vorzugsweise Cycloalkyl und Alkylcycloalkyl, wobei Cyclopentyl und Cyclohexyl bzw. Methyl-cyclopentyl und Methylcyclohexyl besonders bevorzugt sind.

Die Erfindung betrifft außerdem Verfahren zur Herstellung dieser Sulfonylharnstoffe und ihrer physiologisch verträglichen Salze, pharmazeutische Präparate, die diese enthalten oder aus ihnen bestehen sowie die Verwendung zur Behandlung des Diabetes.

Die Verfahren zur Herstellung der Sulfonylharnstoffe und ihrer Salze sind dadurch gekennzeichnet, daß man

a) mit der Gruppe

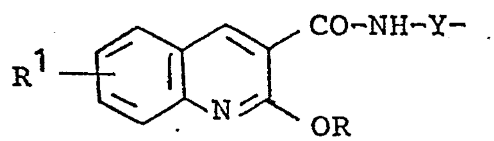

in 4-Stellung substituierte Benzolsulfonylcarbaminsäurederivate mit einem Amin $R^2-NH_2$ oder dessen Salzen umsetzt oder Benzolsulfonamide der Formel

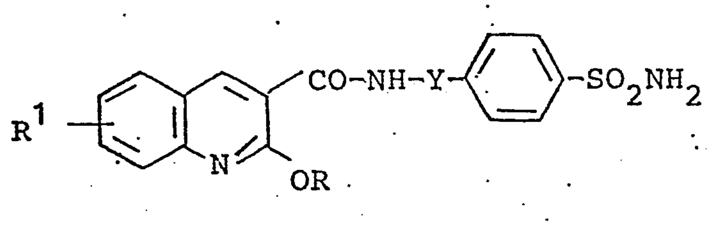

oder deren Salze mit einem $R^2$-substituierten Carbaminsäurederivat umsetzt,

b) mit der Gruppe

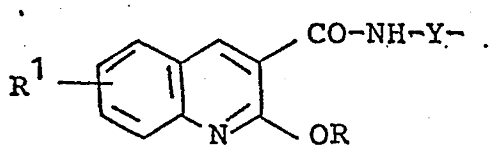

substituierte Benzolsulfonyl-isoharnstoffäther, -isothioharnstoffäther, -parabansäuren oder -halogenameisensäureamidine spaltet,

c) in

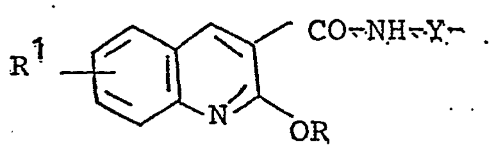

substituierten Benzolsulfonylthioharnstoffen das Schwefelatom durch Sauerstoff ersetzt,

d) entsprechende Benzolsulfinyl- oder -sulfenylharnstoffe oxydiert,

e) in Benzolsulfonylharnstoffe der Formel

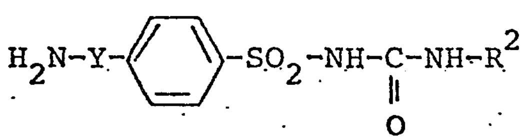

gegebenenfalls stufenweise den Rest

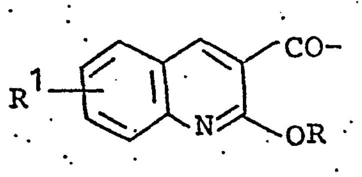

einführt,

f) entsprechend substituierte Benzolsulfonylhalogenide mit $R^2$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Benzolsulfinsäuren oder deren Alkalisalze, mit N-$R^2$-N'-hydroxyharnstoff umsetzt,
erhaltene Salze gegebenenfalls in die freien Verbindungen überführt oder die Reaktionsprodukte gegebenenfalls zur Salzbildung mit alkalischen Mitteln behandelt.

Die in Verfahren a) erwähnten Benzolsulfonylcarbaminsäurederivate sind z.B. Benzolsulfonylcarbaminsäureester, -thiolcarbaminsäureester, -harnstoffe, -semicarbazide oder -semicarbazone. Vorzugsweise wird die Umsetzung von Benzolsulfonylcarbaminsäureestern mit Aminen benutzt.

Die für die Umsetzung der in 4-Stellung substituierten Benzolsulfonamide verwendeten $R^2$-substituierten Carbaminsäurederivate sind z.B. die entsprechenden Isocyanate, Carbaminsäureester, Thiolcarbaminsäureester, Carbaminsäurehalogenide oder Harnstoffe. Vorzugsweise verwendet man hiervon die Reaktion der Sulfonamide mit Isocyanaten bzw. Carbaminsäurechloriden.

Die erwähnten Benzolsulfonyl-carbaminsäureester bzw. -thiolcarbaminsäureester weisen in der Alkoholkomponente einen Alkylrest oder einen Arylrest oder auch einen heterocyclischen Rest auf. Da dieser Rest bei der Reaktion abgespalten wird, hat seine chemische Konstitution keinen Einfluß auf den Charakter des Endproduktes und kann deshalb in weiten Grenzen variiert werden. Das gleiche gilt für die N-$R^2$-substituierten Carbaminsäureester bzw. die entsprechenden Thiolcarbaminsäureester.

Die als Ausgangsstoffe des Verfahrens infrage kommenden Benzolsulfonylharnstoffe sind an der der Sulfonylgruppe abgewandten Seite des Harnstoffmoleküls unsubstituiert oder ein- oder insbesondere zweifach substituiert. Da diese Substituenten bei der Reaktion mit Aminen abgespalten werden, kann ihr Charakter in weiten Grenzen variiert werden. Neben alkyl-, aryl-, acyl- oder heterocyclisch substituierten Benzolsulfonylharnstoffen eignen sich auch Benzolsulfonylcarbamoylimidazole und ähnliche Verbindungen oder Bisbenzolsulfonylharnstoffe, die an einem der Stickstoffatome noch einen weiteren Substituenten z.B. Methyl, tragen können. Man behandelt beispielsweise derartige Bis-(benzolsulfonyl)-harnstoffe oder auch N-Benzolsulfonyl-N'-acylharnstoffe mit $R^2$-substituierten Aminen und erhitzt die erhaltenen Salze auf erhöhte Temperaturen, insbesondere solche oberhalb 100°C.

Weiterhin eignen sich $R^2$-substituierte Harnstoffe oder solche $R^2$-substituierten Harnstoffe, die am freien Stickstoffatom noch ein- oder insbesondere zweifach substituiert sind als Ausgangsverbindungen zur Umsetzung mit

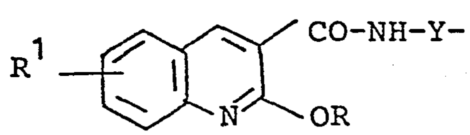

in 4-Stellung substituierten Benzolsulfonamiden. Als solche Ausgangsstoffe kommen beispielsweise infrage N-Cyclohexylharnstoff, die entsprechenden N'-Acetyl, N'-Nitro, N'-Cyclohexyl-, N',N'-Diphenyl- (wobei die beiden Phenylreste auch substituiert sowie direkt oder auch über ein Brückenglied wie $-CH_2-$, -NH-, -O- oder -S- miteinander verbunden sein können), N'-Methyl-N'-phenyl-, N',N'-Dicyclohexylharnstoffe sowie Cyclohexyl-carbamoylimidazole, -pyrazole oder -triazole sowie solche der genannten Verbindungen, die anstelle des Cyclohexyls einen anderen im Bereich der Definition für $R^2$ liegenden Substituenten tragen.

Die Spaltung der als Ausgangsstoffe in Verfahren b) genannten Benzolsulfonylparabansäuren, -isoharnstoffäther, -isothioharnstoffäther oder -halogenameisensäureamidine erfolgt zweckmäßig in Gegenwart von Basen. Isoharnstoffäther können auch in einem sauren Medium mit gutem Erfolg gespalten werden.

Der Ersatz des Schwefelatoms in der Thioharnstoffgruppierung von entsprechend substituierten Benzolsulfonylthioharnstoffen durch ein Sauerstoffatom erfolgt in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten. Auch durch Behandlung mit Phosgen oder Phosphorpentachlorid werden Thioharnstoffe entschwefelt. Als Zwischenstufe erhaltene Chlorameisensäureamidine bzw. Carbodiimide werden durch geeignete Maßnahmen wie Verseifen oder Anlagerung von Wasser in die Benzolsulfonylharnstoffe überführt.

Die Oxidation von Benzolsulfinyl- bzw. Benzolsulfenylharnstoffen erfolgt nach an sich bekannter Methode, vorzugsweise mit Oxydationsmitteln wie Permanganat oder Wasserstoffperoxid.

Die Acylierung der Sulfonylharnstoffe gemäß Verfahren e) wird mit reaktiven Derivaten der Säure

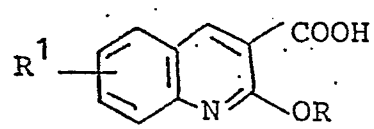

wie beispielsweise Halogeniden, gemischten Anhydriden oder Aktivestern durchgeführt.

Als Sulfonyl- bzw. Sulfinylhalogenide gemäß Verfahren f) eignen sich insbesondere die Chloride. Als saures Kondensationsmittel setzt man beispielsweise Thionylchlorid oder Polyphosphorsäure ein.

Die Herstellung der physiologisch verträglichen Salze erfolgt nach an sich bekannten Methoden. Zur Salzbildung sind insbesondere geeignet Alkali- und Erdalkalihydroxyde, -carbonate oder-bicarbonate sowie physiologisch verträgliche organische Basen.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise werden die Umsetzungen in Abwesenheit oder Anwesenheit von Lösungsmitteln, bei Raumtemperatur oder bei erhöhter Temperatur durchgeführt.

Je nach dem Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthese wenig geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die als Ausgangsstoffe dienenden 2-OR-substituierten Chinolin-3-carbonsäuren sind literaturbekannt oder können nach beschriebenen Verfahren hergestellt werden (vergl. z.B. Beilstein 22, 232).

Die erhaltenen Verbindungen werden vorzugsweise durch Umfällen und/oder Umkristallisieren gereinigt. Eine andere Methode zur Reinigung besteht darin, daß man die Substanz aus einem kristallinen (Alkali)-Salz in einem geeigneten Lösungsmittel in Freiheit setzt.

Die erfindungsgemäßen Verbindungen zeichnen sich durch wertvolle pharmakologische Eigenschaften, insbesondere blutzuckersenkende, aus. Sie eignen sich daher als Arzneimittel, insbesondere als Antidiabetika.

Die blutzuckersenkende Wirkung der erfindungsgemäßen Benzolsulfonylharnstoffe wird beispielsweise dadurch festgestellt, daß man sie als freie Verbindungen oder in Form der Natriumsalze an normal ernährte Kaninchen verfüttert und den Blutzuckerwert nach der bekannten Methode von Hagedorn-Jensen oder mit einem Autoanalyzer über eine längere Zeitdauer ermittelt.

Die routinemäßige Bestimmung der blutzuckersenkenden Wirkung erfolgt z.B, mit Dosierungen von z.B. 10 mg oder 2 mg oder 0,4 mg Wirksubstanz pro kg Versuchstier nach bekannten Methoden.

Die folgenden Verbindungen I bis III wurden in Dosierungen von 2 mg/kg Kaninchen oral verabreicht und die Blutzuckerwerte wurden mit einem Autoanalyzer über eine längere Zeitdauer ermittelt. Die hierbei gemessene Blutzuckersenkung ist in der nachfolgenden Tabelle in % ... Stunden angegeben.

I N-(4-⟨2-(2-Methoxy-chinolin-3-carboxamido)-äthyl⟩-benzolsulfonyl)-N*-cyclopentyl-harnstoff

II N-(4-⟨2-(2-Äthoxy-chinolin-3-carboxamido)-äthyl⟩-benzolsulfonyl)-N*-(3-methyl-cyclopentylmethyl)-harnstoff-K-Salz

III N-(4-⟨2-(2-Phenoxy-chinolin-3-carboxamido)-äthyl⟩-benzolsulfonyl)-N*-butyl-harnstoff

Tabelle

| Verbindung | Blutzuckersenkung am Kaninchen nach Verabreichung von 2 mg/kg p.o. in % nach 1 | 3 | 5 | 24 Stunden |
|---|---|---|---|---|
| I | -21 | -31 | -35 | -11 |
| II | -29 | -27 | -23 | |
| III | -27 | -31 | -42 | |

Die Eigenschaften der Verbindungen erlauben es, in der Therapie des Diabetes mellitus mit so geringen Dosen auszukommen, daß das Präparat nur die verminderte Ansprechbarkeit des Pankreas auf einen erhöhten Blutzuckerspiegel wieder normalisiert.

Die erfindungsgemäßen Sulfonylharnstoffe dienen vorzugsweise zur Herstellung von oral verabreichbaren Präparaten zur Behandlung des Diabetes mellitus. Sie werden als solche oder in Form ihrer Salze bzw. in Gegenwart von Stoffen, die zu einer Salzbildung führen, appliziert. Zur Salzbildung werden beispielsweise alkalische Mittel wie Alkali- oder Erdalkalihydroxyde, -carbonate oder-bicarbonate herangezogen. Die Präparate können neben dem Sulfonylharnstoff bzw. dessen Salz auch noch andere Wirkstoffe enthalten. Als medizinische Präparate kommen vorzugsweise Tabletten in Betracht, die neben den Sulfonylharnstoffen oder deren Salzen die üblichen Träger- und Hilfsstoffe wie Talkum, Stärke, Milchzucker oder Magnesiumstearat enthalten. Dabei kann es zweckmäßig sein, den oder die Wirkstoffe in gemahlener oder fein verteilter Form oder als Gemisch dieser Formen einzusetzen. Ein Präparat, das die erfindungsgemäßen Benzolsulfonylharnstoffe als Wirkstoff enthält, z.B. eine Tablette oder ein Pulver mit oder ohne Zusätze, ist zweckmäßig in eine geeignet dosierte Form

0089597

gebracht. Als Dosis ist dabei eine solche zu wählen, die der Wirksamkeit des verwendeten Benzolsulfonylharnstoffs und dem gewünschten Effekt angepaßt ist. Zweckmäßig beträgt die Dosierung je Einheit etwa 0,5 bis 50 mg, vorzugsweise 1 bis 20 mg, jedoch können auch darüber oder darunter liegende Dosierungseinheiten verwendet werden, die gegebenenfalls vor Applikation zu teilen bzw. vervielfachen sind.

Die nachfolgenden Beispiele zeigen einige der zahlreichen Verfahrensvarianten, die zur Synthese der erfindungsgemäßen Sulfonylharnstoffe geeignet sind. Sie sollen jedoch nicht eine Einschränkung des Erfindungsgegenstandes darstellen.

Beispiel 1:

N-(4-< 2-(2-Methoxy-chinolin-3-carboxamido)-äthyl > -benzolsulfonyl)-N*-cyclohexyl-harnstoff

2,9 g 4-(2-< 2-Methoxy-chinolin-3-carboxamido)-äthyl > -benzolsulfonamid (Schmp. 210 - 212$^{o}$C, hergestellt durch Reaktion von 2-Methoxy-chinolin-3-carbonsäure mit Chlorameisensäuremethylester und Triäthylamin und+) 4-(2-Aminoäthyl)-benzolsulfonamid) werden in 150 ml Butanon-2 nach Zusatz von 2,07 g gemahlener Pottasche und 1,03 g Cyclohexylisocyanat 5 Stunden unter Rückfluß gerührt. Nach dem Abkühlen saugt man die Kaliumsalze ab, suspendiert in Wasser und säuert unter Rühren mit verdünnter Essigsäure an. Der Niederschlag wird erneut abgesaugt und anschließend aus Äthanol/Dimethylformamid umkristallisiert. Der erhaltene N-(4-< 2-(2-Methoxy-chinolin-3-carboxamido)-äthyl > -benzolsulfonyl)-N*-cyclohexyl-harnstoff schmilzt bei 201 - 203$^{o}$C.

+) anschließende Umsetzung mit

In analoger Weise erhält man

N-(4-< 2-(2-Methoxy-chinolin-3-carboxamido)-äthyl > -benzolsulfonyl)-N*-(4-methylcyclohexyl)-harnstoff
vom Schmp. 227 - 229$^{o}$C (aus Äthanol-Dimethylformamid)

N-(4-< 2-(2-Methoxy-chinolin-3-carboxamido)-äthyl > -benzolsulfonyl)-N*-butyl-harnstoff
vom Schmp. 175 - 177$^{o}$C (aus Äthanol)

N-(4-< 2-(2-Methoxy-chinolin-3-carboxamido)-äthyl > -benzolsulfonyl)-N*-cyclopentyl-harnstoff
vom Schmp. 212 - 214$^{o}$C (aus Nitromethan)

In analoger Weise erhält man aus 4-(2-< 6-Chlor-2-methoxy-chinolin-3-carboxamido)-äthyl > -benzolsulfonamid (Schmp. 199 - 201$^{o}$C) den

N-(4-< 2-(6-Chlor-2-methoxy-chinolin-3-carboxamido)-äthyl > -benzolsulfonyl)-N*-butyl-harnstoff
vom Schmp. 193 - 195°C (aus Äthanol-Dimethylformamid)

Beispiel 2:

N-(4-<2-(2-Äthoxy-chinolin-3-carboxamido)-äthyl>-benzolsulfonyl)-N*-cyclopentyl-harnstoff

3,0 g 4-(2-<2-Äthoxy-chinolin-3-carboxamido>-äthyl)-benzolsulfonamid (Schmp. 200 - 202°C, hergestellt durch Reaktion von 2-Äthoxy-chinolin-3-carbonsäure mit Chlorameisensäuremethylester und Triäthylamin und+) 4-(2-Aminoäthyl)-benzolsulfonamid) werden in 150 ml Butanon-2 nach Zusatz von 2,07 g gemahlener Pottasche und 0,92 g Cyclopentylisocyanat 5 Stunden unter Rückfluß gerührt. Nach dem Abkühlen saugt man die Kaliumsalze ab, suspendiert in Wasser und säuert unter Rühren mit verdünnter Essigsäure an. Man saugt den Niederschlag erneut ab und kristallisiert ihn aus Äthanol-Dimethylformamid und Nitromethan um. Der N-(4-<2-(2-Äthoxy-chinolin-3-carboxamido)-äthyl>-benzolsulfonyl)-N*-cyclopentyl-harnstoff schmilzt bei 198 - 200°C.

+) anschließende Umsetzung mit

In analoger Weise erhält man

N-(4-<2-(2-Äthoxy-chinolin-3-carboxamido)-äthyl>-benzolsulfonyl)-N*-(4-methyl-cyclohexyl)-harnstoff
vom Schmp. 194 - 196°C (aus Äthanol)

N-(4-<2-(2-Äthoxy-chinolin-3-carboxamido)-äthyl>-benzolsulfonyl)-N*-butyl-harnstoff
vom Schmp. 158 - 160°C (aus Äthanol)

N-(4-<2-(2-Äthoxy-chinolin-3-carboxamido)-äthyl>-benzolsulfonyl)-N*-cyclohexyl-harnstoff
vom Schmp. 194 - 196°C (aus Äthanol)

In analoger Weise erhält man aus 4-(2-<2-Butoxy-chinolin-3-carboxamido>-äthyl)-benzolsulfonamid (Schmp. 171 - 173°C) den

N-(4-<2-(2-Butoxy-chinolin-3-carboxamido)-äthyl>-benzolsulfonyl)-N*-cyclohexyl-harnstoff vom Schmp. 168 - 170°C (aus Äthanol)

N-(4-<2-(2-Butoxy-chinolin-3-carboxamido)-äthyl>-benzolsulfonyl)-N*-butyl-harnstoff vom Schmp. 143 - 145°C (aus Äthanol)

Beispiel 3:

N-(4-<2-(2-Pentoxy-chinolin-3-carboxamido)-äthyl>-benzolsulfonyl)-N*-butyl-harnstoff

2,64 g 4-(2-<2-Pentoxy-chinolin-3-carboxamido>-äthyl)-benzolsulfonamid (Schmp. 172 - 173°C, hergestellt durch Reaktion von 2-Pentoxy-chinolin-3-carbonsäure mit Chlorameisensäuremethylester und Triäthylamin und+) 4-(2-Aminoäthyl)-benzolsulfonamid) werden analog Beispiel 1 umgesetzt. Nach dem Aufarbeiten und Umkristallisieren aus Äthanol schmilzt der N-(4-<2-(2-Pentoxy-chinolin-3-carboxamido)-äthyl>-benzolsulfonyl)-N*-butyl-harnstoff bei 143 - 145°C.

+) anschließende Umsetzung mit

In analoger Weise erhält man

N-(4-<2-(2-Pentoxy-chinolin-3-carboxamido)-äthyl>-benzolsulfonyl)-N*-(4-methyl-cyclohexyl)-harnstoff vom Schmp. 200 - 202°C (aus Äthanol)

In analoger Weise erhält man aus 4-(2-<2-Isopentoxy-chinolin-3-carboxamido>-äthyl)-benzolsulfonamid (Schmp. 160 - 161°C) den

N-(4-< 2-(2-Isopentoxy-chinolin-3-carboxamido)-äthyl> -benzolsulfonyl)-N*-cyclohexyl-harnstoff
vom Schmp. 179 - 181°C (aus Äthanol)

N-(4-< 2-(2-Isopentoxy-chinolin-3-carboxamido)-äthyl> -benzolsulfonyl)-N*-butyl-harnstoff
vom Schmp. 164 - 166°C (aus Äthanol)

In analoger Weise erhält man aus 4-(2-< 2-Cyclohexyloxy-chinolin-3-carboxamido> -äthyl)-benzolsulfonamid (Schmp. 179 - 181°C) den

N-(4-< 2-(2-Cyclohexyloxy-chinolin-3-carboxamido)-äthyl> -benzolsulfonyl)-N*-(4-methyl-cyclohexyl)-harnstoff
vom Schmp. 188 - 190°C (aus Nitromethan)

N-(4-< 2-(2-Cyclohexyloxy-chinolin-3-carboxamido)-äthyl> -benzolsulfonyl)-N*-butyl-harnstoff
vom Schmp. 179 - 181°C (aus Äthanol)

## Beispiel 4:

**N-(4-< 2-(2-Phenoxy-chinolin-3-carboxamido)-äthyl> -benzolsulfonyl)-N*-cyclohexyl-harnstoff**

3,36 g 4-(2-< 2-Phenoxy-chinolin-3-carboxamido> -äthyl)-benzolsulfonamid (Schmp. 280 - 282°C, hergestellt durch Reaktion von 2-Phenoxy-chinolin-3-carbonsäure mit Chlorameisensäuremethylester und Triäthylamin und+) 4-(2-Aminoäthyl)-benzolsulfonamid) werden mit Cyclohexylisocyanat analog Beispiel 1 umgesetzt. Nach dem Aufarbeiten und Umkristallisieren aus Äthanol schmilzt der N-(4-< 2-(2-Phenoxy-chinolin-3-carboxamido)-äthyl> -benzolsulfonyl)-N*-cyclohexyl-harnstoff bei 190 - 192°C.

+) anschließende Umsetzung mit

In analoger Weise erhält man den

N-(4-⟨2-(2-Phenoxy-chinolin-3-carboxamido)-äthyl⟩-benzolsulfonyl)-N*-butyl-harnstoff vom Schmp. 160 - 162°C (aus Äthanol)

In analoger Weise erhält man aus 4-(2-⟨2-(4-Tolyloxy)-chinolin-3-carboxamido⟩-äthyl)-benzolsulfonamid (Schmp. 178 - 180°C) den

N-(4-⟨2-(2-⟨4-Tolyloxy⟩-chinolin-3-carboxamido)-äthyl⟩-benzolsulfonyl)-N*-butyl-harnstoff vom Schmp. 198 - 200°C (aus Äthanol)

In analoger Weise erhält man aus 4-(2-⟨2-(4-Methoxy-phenoxy)-chinolin-3-carboxamido⟩-äthyl)-benzolsulfonamid (Schmp. 188 - 190°C) den

N-(4-⟨2-(2-⟨4-Methoxy-phenoxy⟩-chinolin-3-carboxamido)-äthyl⟩-benzolsulfonyl)-N*-cyclohexyl-harnstoff vom Schmp. 173 - 174°C (aus Äthanol-Dimethylformamid)

In analoger Weise erhält man aus 4-(2-⟨2-(4-Chlor-phenoxy)-chinolin-3-carboxamido⟩-äthyl)-benzolsulfonamid (Schmp. 219 - 220°C) den

N-(4-⟨2-(2-⟨4-Chlor-phenoxy⟩-chinolin-3-carboxamido)-äthyl⟩-benzolsulfonyl)-N*-cyclohexyl-harnstoff vom Schmp. 207 - 209°C (aus Äthanol)

## Beispiel 5:

**N-(4-⟨2-(2-Methoxy-chinolin-3-carboxamido)-äthyl⟩-benzolsulfonyl)-N*-(3-methyl-cyclopentylmethyl)-harnstoff-K-Salz**

Wie in Beispiel 1 beschrieben, wird die Reaktion mit dem dort erwähnten Sulfonamid und 3-Methyl-cyclopentylmethylisocyanat durch-

geführt. Nach dem Absaugen der Kaliumsalze werden diese in Wasser suspendiert und die Mischung mit Essigsäure neutralisiert. Der Niederschlag wird erneut abgesaugt und aus Nitromethan umkristallisiert. Das dabei erhaltene K-Salz schmilzt bei 225 - 227°C.

Beispiel 6:

N-(4-<2-(2-Äthoxy-chinolin-3-carboxamido)-äthyl>-benzolsulfonyl)-N*-(3-methyl-cyclopentylmethyl)-harnstoff-K-Salz

Analog Beispiel 5 wird das in Beispiel 2 beschriebene Sulfonamid umgesetzt. Das so erhaltene K-Salz schmilzt nach dem Umkristallisieren aus Nitromethan bei 212 - 214°C.

Beispiel 7:

N-(4-<2-(2-Pentoxy-chinolin-3-carboxamido)-äthyl>-benzolsulfonyl)-N*-cyclohexyl-harnstoff-K-Salz

Analog Beispiel 5 wird das in Beispiel 3 erwähnte Sulfonamid umgesetzt. Das so erhaltene K-Salz wird aus Äthanol umkristallisiert und schmilzt bei 202 - 204°C.

Patentansprüche:

1. Sulfonylharnstoffe der Formel

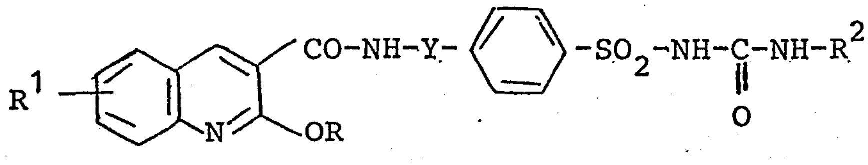

in welcher

Y Alkylen mit 2 - 3 C-Atomen

R Alkyl mit 1 - 8 C-Atomen, Cycloalkyl oder Alkylcycloalkyl mit jeweils 5 - 8 C-Atomen, Phenyl, das ggf. durch Alkyl mit 1 - 4 C-Atomen, Alkoxy mit 1 - 4 C-Atomen im Alkylteil oder Halogen substituiert ist,

$R^1$ Wasserstoff, Chlor, Alkyl mit 1 - 4 C-Atomen

$R^2$ Alkyl mit 3 - 6 C-Atomen, Cycloalkyl, Alkylcycloalkyl, Cycloalkylalkyl, Cycloalkenyl, Alkylcycloalkenyl oder Cycloalkenylalkyl mit jeweils 5 - 8 C-Atomen

und deren physiologisch verträgliche Salze.

2. Verbindungen gemäß Anspruch 1, dadurch gekennzeichnet, daß $R^1$ Wasserstoff, $R^2$ Cyclopentyl, Cyclohexyl, Methylcyclopentyl oder Methylcyclohexyl, R Alkyl mit 1 - 5 C-Atomen, Cyclohexyl oder Phenyl und Y die $-CH_2-CH_2-$Gruppe bedeuten.

3. Verfahren zur Herstellung von Sulfonylharnstoffen gemäß Anspruch 1, dadurch gekennzeichnet, daß man

a) mit der Gruppe

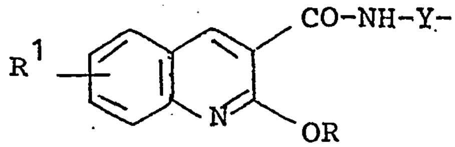

in 4-Stellung substituierte Benzolsulfonylcarbaminsäurederivate mit einem Amin $R^2$-$NH_2$ oder dessen Salzen umsetzt

oder Benzolsulfonamide der Formel

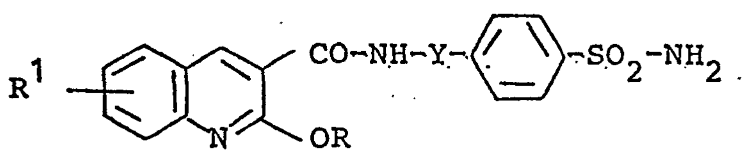

oder deren Salze mit einem $R^2$-substituierten Carbaminsäurederivat umsetzt,

b) mit der Gruppe

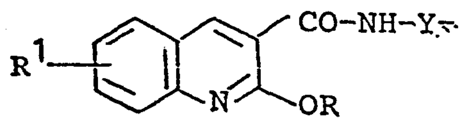

substituierte Benzolsulfonyl-isoharnstoffäther, -isothioharnstoffäther, -parabansäuren oder -halogenameisensäureamidine spaltet,

c) in

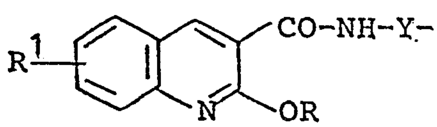

substituierten Benzolsulfonylthioharnstoffen das Schwefelatom durch Sauerstoff ersetzt,

d) entsprechende Benzolsulfinyl- oder-sulfenylharnstoffe oxydiert

e) in Benzolsulfonylharnstoffe der Formel

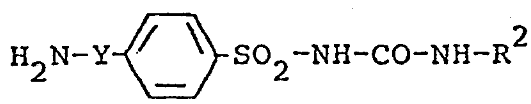

gegebenenfalls stufenweise den Rest

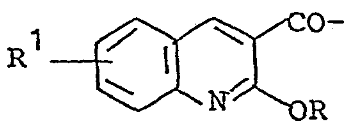

einführt,

f) entsprechend substituierte Benzolsulfonylhalogenide mit $R^2$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Benzolsulfinsäuren oder deren Alkalisalze mit N-$R^2$-N'-hydroxyharnstoff umsetzt,
erhaltene Salze gegebenenfalls in die freien Verbindungen überführt oder die Reaktionsprodukte gegebenenfalls zur Salzbildung mit alkalischen Mitteln behandelt.

4. Arzneimittel auf Basis eines Sulfonylharnstoffs gemäß Anspruch 1 oder eines seiner Salze.

5. Verfahren zur Herstellung eines Arzneimittels gemäß Anspruch 3, dadurch gekennzeichnet, daß man einen Sulfonylharnstoff der im Anspruch 1 angegebenen Formel oder eines seiner Salze in eine geeignete Applikationsform bringt.

6. Verwendung eines Sulfonylharnstoffs gemäß Anspruch 1 oder eines seiner Salze bei der Bekämpfung von Diabetes.

7. Verfahren zur Senkung des Blutzuckerspiegels, dadurch gekennzeichnet, daß man eine wirksame Menge einer Verbindung gemäß Anspruch 1 oral verabreicht.

Patentansprüche für Österreich:

1. Verfahren zur Herstellung von Sulfonylharnstoffen der Formel I

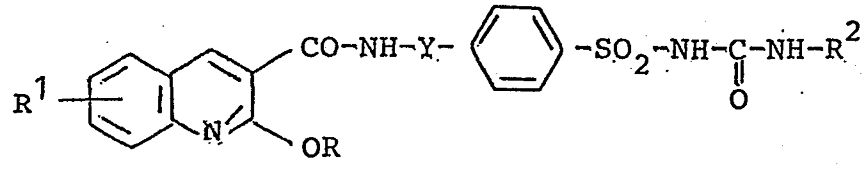

I

in welcher

Y Alkylen mit 2 - 3 C-Atomen

R Alkyl mit 1 - 8 C-Atomen, Cycloalkyl oder Alkylcycloalkyl mit jeweils 5 - 8 C-Atomen, Phenyl, das ggf. durch Alkyl mit 1 - 4 C-Atomen , Alkoxy mit 1 - 4 C-Atomen im Alkylteil oder Halogen substituiert ist,

$R^1$ Wasserstoff, Chlor, Alkyl mit 1 - 4 C-Atomen

$R^2$ Alkyl mit 3 - 6 C-Atomen, Cycloalkyl, Alkylcycloalkyl, Cycloalkylalkyl, Cycloalkenyl, Alkylcycloalkenyl oder Cycloalkenylalkyl mit jeweils 5 - 8 C-Atomen

und von deren physiologisch verträglichen Salzen, dadurch gekennzeichnet, daß man

a) mit der Gruppe

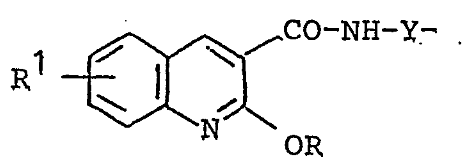

in 4-Stellung substituierte Benzolsulfonylcarbaminsäurederivate mit einem Amin $R^2-NH_2$ oder dessen Salzen umsetzt
oder Benzolsulfonamide der Formel

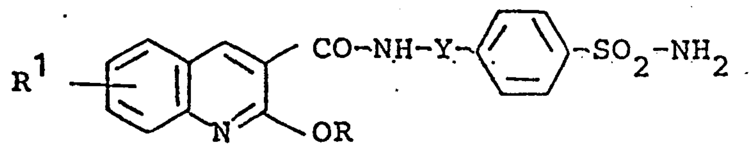

oder deren Salze mit einem $R^2$-substituierten Carbaminsäurederivat umsetzt,

b) mit der Gruppe

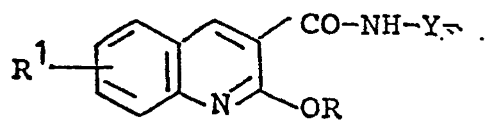

substituierte Benzolsulfonyl-isoharnstoffäther, -isothioharnstoffäther, -parabansäuren oder -halogenameisensäureamidine spaltet,

c) in

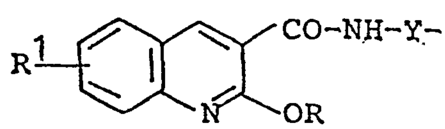

substituierten Benzolsulfonylthioharnstoffen das Schwefelatom durch Sauerstoff ersetzt,

d) entsprechende Benzolsulfinyl- oder-sulfenylharnstoffe oxydiert

e) in Benzolsulfonylharnstoffe der Formel

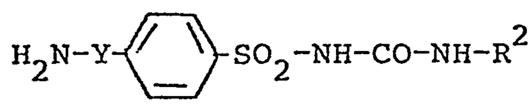

gegebenenfalls stufenweise den Rest

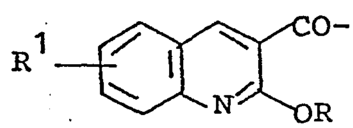

einführt,

f) entsprechend substituierte Benzolsulfonylhalogenide mit $R^2$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Benzolsulfinsäuren oder deren Alkalisalze mit N-$R^2$-N'-hydroxyharnstoff umsetzt,
erhaltene Salze gegebenenfalls in die freien Verbindungen überführt oder die Reaktionsprodukte gegebenenfalls zur Salzbildung mit alkalischen Mitteln behandelt.

2. Verfahren gemäß Anspruch 1, dadurch gekennzeichnet, daß man einen Sulfonylharnstoff der Formel I, worin $R^1$ Wasserstoff, $R^2$ Cyclopentyl, Cyclohexyl, Methylcyclopentyl oder Methylcyclohexyl, R Alkyl mit 1 - 5 C-Atomen, Cyclohexyl oder Phenyl und Y die $-CH_2-CH_2-$Gruppe bedeuten, oder ein physiologisch verträgliches Salz davon herstellt.